# DEMANDE DE BREVET EUROPEEN

(11) **EP 1 120 152 A1**
(43) Date de publication de la demande: **01.08.2001**
(21) Numéro de dépôt: 01400004.6
(22) Date de dépôt: 03.01.2001
(51) Int. Cl.: B01F 3/02, B01F 3/00

(54) **Procédé et dispositif pour l'optimisation de mélanges de gaz réactifs**

(30) Priorité: 25.01.2000 FR 0000946
(71) Demandeur: L'AIR LIQUIDE, SOCIETE ANONYME POUR L'ETUDE ET L'EXPLOITATION DES PROCEDES GEORGES CLAUDE, 75321 Paris Cédex 07 (FR)
(72) Inventeur: Bockel-Macal, Savine, 75321 Paris Cedex 07 (FR); Illy, Fabien, 75321 Paris Cedex 07 (FR); Avrillier, Pierre, 75321 Paris Cedex 07 (FR)
(74) Mandataire: Ducreux, Marie

(57) **Abrégé**

L'invention concerne un procédé pour déterminer le risque d'inflammabilité du mélange de deux gaz réactifs A, B, dans un gaz inerte ou de fond, caractérisé en ce qu'il comporte :
- une étape de détermination, dans le diagramme d'inflammabilité du mélange A-B-gaz de fond, de l'évolution de la composition du mélange, pour déterminer si la zone d'inflammabilité dudit diagramme d'inflammabilité est traversée ou pas, lorsque A est d'abord injecté dans le gaz inerte ou de fond pour former un premier mélange, puis B est injecté dans le premier mélange pour former le mélange final,
- une étape de comparaison du temps de mélange, en cas de traversée du domaine, au temps d'induction chimique du mélange.

## Description

### Domaine technique et art antérieur

L'invention concerne la réalisation de mélanges de gaz potentiellement réactifs, ainsi que la détermination de schémas de mélanges de tels gaz.

Pour réaliser en toute sécurité des mélanges de gaz potentiellement réactifs, diverses méthodes et/ou appareils de mélange sont actuellement proposés, qui prennent en compte la capacité du mélange en statique ; c'est-à-dire que seule la qualité du mélange final est considérée.

C'est par exemple le cas du document US-5 801 265, qui concerne un procédé d'injection d'oxygène dans un réactant, pour lequel un dispositif de contrôle assure un mélange d'oxygène et de réactants de composition finale située au-dessus d'une certaine limite supérieure d'inflammabilité.

Les études sur les mélanges de gaz dans les brûleurs sont en général orientées vers la réaction des gaz et en particulier vers les produits de combustion qui doivent respecter un cahier des charges précis (en particulier : formation réduite de NOx, limitation de la température maximale).

On ne connaît actuellement pas de méthode permettant de déterminer un schéma d'injection de gaz en prenant en compte l'évolution de la composition du mélange au cours de sa réalisation.

### Exposé de l'invention

L'invention propose une autre approche afin de déterminer les possibilités de réaliser un mélange gazeux potentiellement inflammable.

Plus précisément, l'invention propose d'étudier la mise en oeuvre d'un mélange de gaz réactifs, en prenant en compte le phénomène de mélange proprement dit, c'est-à-dire les risques liés à la réactivité qui peuvent apparaître au cours de la phase de mélange.

L'invention concerne notamment un procédé pour déterminer le risque d'inflammabilité du mélange d'au moins deux gaz réactifs A, B, dans un gaz inerte ou un gaz de fond (gaz pouvant contenir les gaz A, B et d'autres espèces, réactives ou non), ou pour déterminer l'ordre de mélange des gaz réactifs dans le gaz inerte ou de fond.

On entend notamment par gaz inerte un gaz ne conduisant pas, dans les conditions du mélange, à des réactions chimiques avec A ou B ou avec lui-même.

Des modes de formation de mélange (ou modes de mélange) sont définis, suivant l'ordre dans lequel les gaz réactifs sont successivement mélangés dans le gaz inerte ou de fond: selon un premier mode, le gaz A est d'abord mélangé dans un gaz inerte ou de fond, formant avec celui-ci un premier mélange dans lequel le gaz B est ensuite mélangé ; selon un second mode, le gaz B est d'abord mélangé dans le gaz inerte ou de fond, formant avec celui-ci un premier mélange dans lequel le gaz A est ensuite mélangé. Si le mélange met en oeuvre plus de deux gaz dans le gaz inerte ou de fond, d'autres modes de formation de mélange peuvent être définis.

A partir du diagramme ternaire du mélange considéré (qu'il s'agisse d'un mélange de deux ou de plusieurs gaz dans le gaz inerte ou le gaz de fond), on peut déterminer l'évolution de la composition du mélange lorsque A est d'abord injecté dans le gaz inerte, ou le gaz de fond, pour former un premier mélange, puis B est injecté dans le premier mélange pour former le mélange final.

La prise en compte du diagramme ternaire assure la connaissance directe de la composition du mélange par rapport à sa zone d'inflammabilité, ce qui n'est pas le cas si l'on utilise des diagrammes binaires.

Si la composition du mélange évolue dans le diagramme ternaire sans traverser la zone d'inflammabilité, ce mode d'injection peut être retenu, car son risque d'inflammabilité est nul.

On peut également comparer, pour l'un et/ou l'autre des modes de mélange, le temps de traversée, par le mélange et pendant la constitution de celui-ci, de la zone d'inflammabilité de ce diagramme, ou le temps de mélange (qui est supérieur au temps de traversée) au temps d'induction chimique de ce même mélange.

Le temps de mélange est lié à la technologie du mélangeur. Il dépend, en effet, des vitesses de l'écoulement généré par le mélangeur et de sa capacité à mélanger rapidement.

L'invention a donc pour objet un procédé pour déterminer le risque d'inflammabilité du mélange d'au moins deux gaz réactifs A, B, dans un gaz inerte ou de fond, ou l'ordre de mélange de ces gaz réactifs dans le gaz inerte ou de fond, caractérisé en ce qu'il comporte :
- une étape pour déterminer si la composition du mélange traverse, pendant sa formation, la zone d'inflammabilité dans le diagramme ternaire du mélange A-B-gaz de fond (ou inerte), lorsque ce mélange est réalisé suivant un premier mode selon lequel A est d'abord mélangé dans le gaz inerte ou de fond pour former un premier mélange, puis B est mélangé dans le premier mélange pour former le mélange final.

Ce procédé peut en outre comporter :
- une étape de détermination d'un premier temps, de traversée de la zone d'inflammabilité dudit diagramme ternaire, ce temps de traversée dépendant du mélangeur utilisé au cours de la phase de réalisation du mélange qui impose de traverser la zone d'inflammabilité;
- une étape de comparaison de ce premier temps de traversée au temps d'induction chimique du mélange ; ce dernier temps dépend de la réactivité du mélange.

Selon une variante, c'est le temps de mélange, caractéristique du mélangeur utilisé au cours de la phase de réalisation du mélange qui impose de traverser la zone d'inflammabilité, qui est comparé au temps d'induction chimique du mélange.

Si la composition du mélange traverse la zone d'inflammabilité dudit diagramme ternaire, ou bien si le temps de mélange pris en compte ou le premier temps de traversée est supérieur au temps d'induction chimique du mélange les étapes suivantes peuvent aussi être mises en oeuvre:
- une étape pour déterminer si la composition du mélange traverse, pendant sa formation, la zone d'inflammabilité dans le diagramme ternaire du mélange A-B-gaz de fond (ou inerte), lorsque celui-ci est réalisé suivant un second mode selon lequel B est d'abord mélangé dans le gaz inerte ou de fond pour former un premier mélange, puis A est mélangé dans le premier mélange pour former le mélange final, et éventuellement :
- une étape de comparaison du temps de mélange du mélangeur, utilisé au cours de la phase de réalisation du mélange qui impose de traverser la zone d'inflammabilité, avec le temps d'induction chimique du mélange lorsque B est d'abord mélangé dans le gaz inerte ou de fond pour former un premier mélange, puis A est mélangé dans le premier mélange pour former le mélange final ; ou bien:
- la détermination d'un deuxième temps de traversée de la zone d'inflammabilité dudit diagramme ternaire, lorsque B est d'abord injecté dans le gaz inerte ou de fond pour former un premier mélange, puis A est injecté dans le premier mélange pour former le mélange final, et une étape de comparaison de ce deuxième temps de traversée au temps d'induction chimique du mélange.

De préférence, c'est le temps d'induction chimique minimum que l'on compare au temps de mélange. Le temps d'induction pour le mélange stoechiométrique est, en général, proche de ce temps d'induction minimum et peut être pris comme une approximation de ce temps minimum.

Le procédé selon l'invention prend donc en compte le phénomène de mélange. Il permet, à partir des caractéristiques d'inflammabilité du mélange gazeux composé de A, de B et du gaz inerte ou de fond, et des caractéristiques des mélangeurs utilisés, d'évaluer le risque d'inflammabilité au cours de la réalisation du mélange de A dans B, ce dernier étant déjà mélangé avec le gaz inerte ou de fond, ou de B dans A, ce dernier étant déjà mélangé avec le gaz inerte ou de fond, plus que de simplement savoir si le mélange final A+B+gaz inerte ou de fond serait, ou non, inflammable.

L'invention permet également d'évaluer la solution la plus judicieuse et la plus sécuritaire pour savoir quel fluide mélanger en priorité, et pour savoir comment réaliser le mélange. C'est notamment le cas lorsqu'elle est appliquée aux injections de deux fluides réactifs dans un flux majoritairement inerte, comme dans les procédés à recycle.

Le procédé selon l'invention peut s'appliquer à un mélange de plusieurs gaz, le nombre de gaz à mélanger étant supérieur à trois.

Un procédé de mélange de deux gaz réactifs A, B dans un gaz inerte ou de fond, comporte donc :
- la détermination du risque d'inflammabilité du mélange, lors de la réalisation du mélange, ou de l'ordre d'injection des gaz réactifs, selon le procédé conforme à l'invention et décrit ci-dessus,
- le mélange des gaz réactifs A et B, dans l'ordre pour lequel la composition du mélange ne traverse pas la zone d'inflammabilité lors de son évolution, ou pour lequel le, ou les, temps de mélange ou le temps de traversée de la zone d'inflammabilité du diagramme ternaire est (sont) inférieur au temps d'induction chimique du mélange.

Selon l'invention, le cas où on mélange au moins trois gaz réactifs ou combustibles, à un gaz inerte ou de fond peut se ramener au traitement successif de plusieurs cas de mélange de deux gaz à un gaz inerte ou de fond.

La mise en oeuvre de l'invention peut être assurée à l'aide de la consultation de bases de données électroniques.

L'invention concerne également un procédé de réalisation d'une installation pour mélanger au moins deux gaz réactifs A, B, dans un gaz inerte ou de fond, comportant les étapes suivantes:
- on détermine l'ordre de mélange de ces gaz, selon un procédé tel que décrit ci-dessus,
- on réalise une installation, de manière à mélanger les gaz selon l'ordre ainsi déterminé.

Ainsi, des mélangeurs peuvent être disposés sur une canalisation, qui amène le gaz inerte ou de fond, de manière à ce que le mélange des gaz soit fait dans des conditions de sécurité élevées.

L'invention permet donc de réaliser, avec une grande sécurité, une installation pour mélanger au moins deux gaz réactifs A, B, dans un gaz inerte ou de fond.

L'invention concerne également un dispositif pour la mise en oeuvre de l'invention caractérisé en ce qu'il comporte :
- des moyens mémorisant au moins une base de données comportant, pour des mélanges de gaz, des données sur les diagrammes ternaires de ces mélanges, et les zones d'inflammabilité dans ces diagrammes pour des conditions de température et de pression données,
- des moyens pour sélectionner un mélange de gaz et des conditions de pression et de température d'utilisation de ce mélange de gaz,
- des moyens de visualisation d'un diagramme ternaire et de la zone d'inflammabilité d'un mélange dans ce diagramme.

Selon un autre aspect, l'invention a également pour objet un dispositif pour établir le risque d'inflammabilité de mélanges qui sont chacun constitués d'au moins deux gaz réactifs A, B dans un gaz inerte ou de fond, ou pour établir l'ordre de mélange de ces gaz réactifs dans le gaz inerte ou de fond, caractérisé en ce qu'il comporte des moyens pour calculer ou spécialement programmés pour calculer, en fonction de conditions de température et de pression:
- le diagramme ternaire d'un mélange et la zone d'inflammabilité dans ce diagramme,
- un temps d'induction chimique de ces mélanges.

Ce dernier dépend de la température, de la pression, et de la concentration.

L'invention a enfin également pour objet un dispositif terminal pour établir le risque d'inflammabilité du mélange d'au moins deux gaz réactifs A, B, dans un gaz inerte ou de fond, ou l'ordre de mélange de ces gaz réactifs dans le gaz inerte ou de fond, caractérisé en ce qu'il comporte :
- des moyens de communication pour établir une communication entre ledit dispositif terminal et des moyens contenant au moins une base de données comportant, pour des mélanges de gaz, des données sur les diagrammes ternaires de ces mélanges, et les zones d'inflammabilité dans ces diagrammes en fonction des conditions de température et de pression,
- des moyens pour fournir audit terminal des données d'utilisateur dudit terminal, comportant au moins un mélange de gaz utilisé, et les conditions de pression et de température d'utilisation, et éventuellement un ou plusieurs types de mélangeurs déterminés,
- des moyens de mémorisation, en communication avec les moyens pour fournir audit terminal des données d'utilisateur, pour mémoriser ces données d'utilisateur, ainsi que des données fournies par la base de données sur le diagramme ternaire du mélange sélectionné,
- des moyens d'affichage, en communication avec les moyens de mémorisation, pour afficher au moins le diagramme ternaire fourni par la base de données, et éventuellement le temps de mélange d'un mélangeur déterminé.

L'invention permet, par consultation de bases de données, la constitution d'un ensemble d'informations décrivant un comportement d'un mélange de gaz.

Ainsi, le schéma d'une installation destinée au mélange de gaz peut être très rapidement établi, ce qui raccourcit la durée de conception de cette installation.

### Brève description des figures

Les caractéristiques et avantages de l'invention apparaîtront mieux à la lumière de la description qui va suivre. Cette description porte sur les exemples de réalisation, donnés à titre explicatif et non limitatif, en se référant à des dessins annexés sur lesquels :
- la figure 1 représente un organigramme d'un procédé selon l'invention,
- Les figures 2A, 3A et 4A représentent divers schémas de mélange d'un gaz combustible et d'un gaz oxydant dans un gaz de recycle,
- Les figures 2B, 3B, 4B, 5A-6B représentent des diagrammes d'inflammabilité de mélanges combustible-oxydant-gaz inerte,.
- La figure 7A représente un autre schéma de mélange de gaz,
- Les figures 7B et 7C représentent des diagrammes d'inflammabilité de mélanges combustibles-oxydant-gaz inerte,
- Les figures 8 à 9B représentent un exemple de dispositif pour la mise en oeuvre de l'invention.

### Description détaillée de modes de réalisation de l'invention

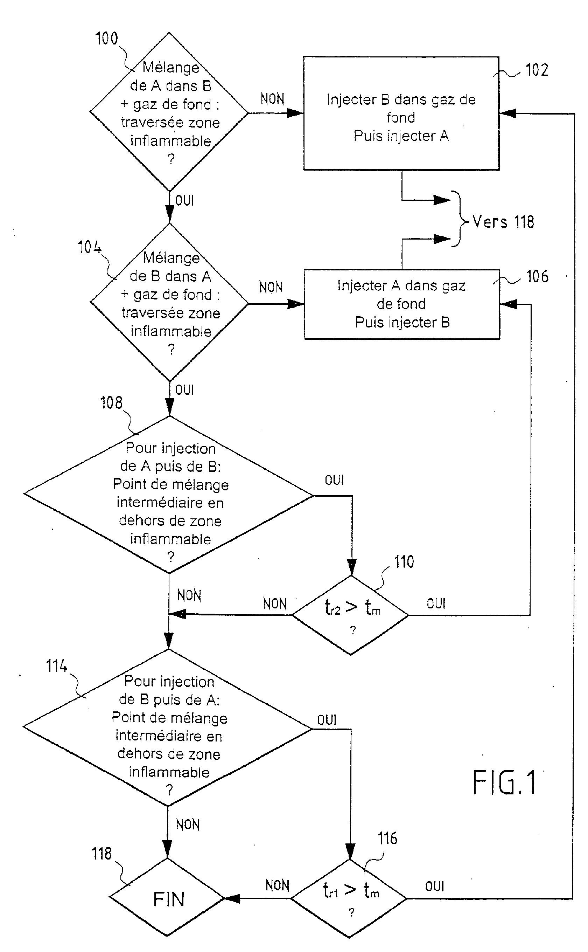

Des étapes de procédés selon l'invention vont être expliquées en liaison avec la figure 1.

Cette figure concerne le cas d'un mélange de deux gaz A et B dans un gaz de fond, par exemple un gaz inerte.

Selon l'invention, on détermine déjà (étape 100) si, pendant la réalisation du mélange de A dans B (lui-même déjà mélangé à un gaz inerte), ce dernier présente un caractère inflammable.

En d'autres termes, on détermine si le domaine d'inflammabilité du diagramme d'inflammabilité du mélange ternaire A-B-gaz de fond (diagramme ternaire) est traversé, pendant la constitution du mélange, lorsque :
- on mélange d'abord B dans le gaz de fond, à l'aide d'un premier mélangeur pour former un premier mélange,
- puis, à l'aide d'un second mélangeur, on mélange A dans le premier mélange obtenu pour former le mélange final.

Ce domaine d'inflammabilité dans le diagramme ternaire peut être par exemple déterminé en utilisant la Loi Le Chatelier, qui permet de tracer les domaines d'inflammabilité à partir de mélanges de combustibles. On peut se reporter à ce sujet à Kuchta, Bulletin 680, Bureau of Mines, 1985.

Si le domaine d'inflammabilité n'est pas traversé, on peut retenir l'ordre d'injection suivant comme ordre à mettre en oeuvre (étape 102) :
- B est d'abord mélangé dans le gaz de fond (premier mélange)
- A est ensuite mélangé dans le premier mélange.

Pour cet ordre de mélange, en effet, le risque d'inflammabilité est nul.

Si, au contraire, ce premier mode de mélange nécessite de traverser la zone d'inflammabilité, on considère (étape 104) l'autre possibilité de former le mélange (second mode), à savoir :
- mélange de A dans le gaz de fond (premier mélange), à l'aide d'un premier mélangeur,
- puis, à l'aide d'un second mélangeur, mélange de B dans le premier mélange.

Si ce second mode de mélange des gaz A et B n'impose pas de traverser le domaine d'inflammabilité (étape 104), alors ce second mode est retenu (étape 106), le risque d'inflammabilité étant nul.

Si les deux modes ainsi définis imposent tous deux de traverser le domaine d'inflammabilité, alors il y a risque d'inflammabilité. Ce risque est cependant maîtrisé si l'ordre de mélange retenu est celui qui répond aux deux critères suivants:
(i) - le point de mélange intermédiaire (premier mélange) est situé en dehors du domaine d'inflammabilité; ce premier critère vise à exclure le cas où le mélange intermédiaire formé présenterait lui-même des caractéristiques d'inflammabilité, situation qui serait très dangereuse et qui présenterait un risque très élevé d'inflammabilité.
(ii) - le temps de mélange du mélangeur utilisé au cours de la phase ou de l'étape qui impose de traverser le domaine d'inflammabilité est inférieur au temps d'induction chimique ou d'auto-inflammation du mélange. Ce second critère est en fait une comparaison entre la réactivité du mélange et la performance du mélangeur (temps de mélange).

La réactivité du mélange est caractérisée par un temps d'induction ou délai d'inflammation : aussi bien avant l'établissement du régime stationnaire qu'avant l'explosion franche, il s'écoule un certain temps, qu'on appelle période d'induction ou délai d'auto-inflammation suivant qu'il précède une ramification lente ou une réaction explosive. La longueur de ces périodes initiales est variable selon la nature des réactifs mis en jeu.

Plus précisément, lorsque l'on porte un mélange inflammable à une pression et à une température telles qu'il puisse exploser, l'auto-inflammation n'intervient qu'après un certain temps : on désigne ce temps par le terme « délai d'auto-inflammation ». L'ordre de grandeur de ce temps est variable suivant les niveaux de température et de pression initiales. Il dépend également des concentrations des composants du mélange.

Par exemple, le délai d'auto-inflammation de l'heptane dans l'air en proportion stoechiométrique à 12 bar passe de 10 secondes (à 400°C) à 25 ms (à 500°C). Pour l'hydrogène stoechiométrique dans l'air à la pression atmosphérique, le délai d'auto-inflammation passe de 100 ms (à 560°C) à 15 ms (à 630°C) (On peut se reporter à l'ouvrage de A. Van Tiggelen, « Oxydations et Combustions » tome 1, publications de l'Institut Français du pétrole, 1968, éditions Technip). La méthode du courant gazeux, ou méthode par écoulement, aussi appelée méthode du tube concentrique, permet de mesurer expérimentalement des temps d'auto-inflammation supérieurs à 0.5 ms. On peut se reporter, en ce qui concerne cette méthode, à l'ouvrage de Van Tiggelen cité ci-dessus. Le comburant, dilué ou non, et chauffé à la température désirée, s'écoule à travers une conduite thermostatée. A une section donnée, une injection de combustible généralement préchauffé est réalisée.

Selon une autre méthode de détermination, on pose les équations des cinétiques des réactions de combustion mises en jeu et leur résolution est faite numériquement.

Le temps de mélange est, lui, propre à la technologie du mélangeur. Il est déterminé par la connaissance de paramètres tels que le temps de parcours moyen ou le temps de parcours particulaire dans le mélange.

Le temps de mélange est le temps pour atteindre une certaine homogénéité du mélange. Cette homogénéité peut être par exemple représentée par le coefficient de variation, défini par le rapport de l'écart-type de la distribution de concentration à la moyenne de cette distribution, par exemple lorsque la distribution de concentration est pondérée par le débit-masse. On considère qu'un mélange est mélangé lorsque le coefficient de variation est inférieur à 1 % ou à 2 % ou à 5 %.

Le temps de mélange global peut être déterminé expérimentalement par des mesures de concentration (mesures par sonde, tomographie, fluorescence induite par laser) et des mesures de vitesse (vélocimétrie laser doppler, vélocimétrie par image de particules).

Un injecteur est un exemple de mélangeur.

Typiquement, les temps de mélange globaux correspondant à l'injecteur décrit dans le brevet US 5 356 213, varient de 100 à 500 ms.

La connaissance détaillée des champs de vitesse et de concentration permet d'obtenir des temps de mélange particulaire à l'aide d'outils numériques (code de mécanique des fluides résolvant les équations de Navier-Stokes).

L'ouvrage intitulé "Measurements Techniques in Fluid Dynamics", Von Karman Institute for Fluid Dynamics, 1994, V.K.I. Library, ISBN : D/1994/0238/417, donne des informations complémentaires sur les techniques de détermination des temps de mélange mentionnées ci-dessus.

Le temps de mélange, pour un état initial et un état final situés sur le diagramme ternaire en dehors de la zone d'inflammabilité, est supérieur au temps de traversée de cette zone par ce mélange.

Par conséquent, selon la figure 1, on détermine, dans le cas où les réponses aux questions posées aux étapes 100 et 104 sont positives, si le point de mélange intermédiaire dans le cas du second mode d'injection, se situe hors de la zone inflammable (étape 108). Si oui, le temps tᵣ₂ de réactivité du mélange pour ce second mode est comparé au temps tₘ, ou temps de mélange, caractéristique du mélangeur utilisé au cours de la phase ou de l'étape qui impose de traverser le domaine d'inflammabilité (étape 110). Si tᵣ₂ > tₘ, le risque d'inflammabilité est maîtrisé et le second mode d'injection peut être retenu à titre de schéma de mélange à réaliser.

Sinon, des étapes similaires (114 et 116) sont mises en oeuvre pour le premier mode d'injection. Le temps tᵣ₁ de réactivité du mélange pour ce premier mode est comparé au temps tₘ, ou temps de mélange, caractéristique du mélangeur utilisé au cours de la phase ou de l'étape qui impose de traverser le domaine d'inflammabilité (étape 116). Si tᵣ₁ > tₘ, le risque est maîtrisé et le premier mode d'injection peut être retenu à titre de schéma de mélange à réaliser.

Il est préférable de comparer le temps de réactivité minimum au temps de mélange.

Dans le cas où le mélange final est un mélange stoechiométrique, on a : tᵣ₁ ≈ tᵣ₂. Autrement dit, dans ce cas, le temps de réactivité est sensiblement le même quel que soit l'ordre dans lequel le mélange est réalisé.

Par ailleurs, le temps de réactivité tᵣₛ du mélange stoechiométrique est, en général, proche ou voisin du temps de réactivité minimum pour le mélange en question.

Si tₘ est comparé avec tᵣₛ et si tₘ < tᵣₛ, alors le mélange peut être réalisé dans un ordre quelconque. Autrement dit, les risques associés aux deux solutions (d'abord B dans le gaz de fond, puis A, ou l'inverse) sont tous deux maîtrisés et on peut choisir un ordre de mélange, ou l'autre.

Si aucun des modes d'injection ne satisfait aux critères imposés, le risque d'inflammabilité n'est plus maîtrisé et le mélange voulu ne peut être réalisé dans des conditions de sécurité suffisantes. L'algorithme est alors terminé (étape 118).

Le schéma de la figure 1 peut aussi être modifié sans sortir du cadre de l'invention, en effectuant les tests successifs sur les modes d'injection dans l'ordre inverse. On peut aussi inverser seulement l'ordre dans lequel on considère les deux modes d'injection au cours des étapes 108 - 110 et 114 - 116.

Selon une autre variante, on peut procéder, dans la deuxième partie du diagramme, c'est-à-dire après l'étape 104, et si il est répondu positivement à la question posée lors de cette étape:
- d'abord, au cours d'une seule et même étape, ou d'un groupe d'étapes successives, à la détermination du mode pour lequel le point intermédiaire se situe hors de la zone inflammable,
- puis, ensuite, au cours d'une seule et même étape, ou d'un groupe d'étapes successives, à la comparaison de la réactivité des deux mélanges (A dans B+inerte et B dans A+inerte) avec la, ou les, performance(s) du, ou des, mélangeur(s).

Selon cette variante, le mélange retenu sera le même que lorsque le procédé est mis en oeuvre selon l'ordre contenu dans le schéma de la figure 1.

Selon la figure 1, on met en oeuvre deux étapes qui permettent de comparer plusieurs modes ou ordres d'injection entre eux :
- première étape : y a-t-il traversée du domaine d'inflammation pendant le mélange? On choisit un ordre ou un mode d'injection des fluides, si cet ordre ou ce mode permet d'éviter la traversée du domaine d'inflammation pendant la constitution du mélange (risque d'inflammabilité nul). Si la constitution du mélange nécessite la traversée du domaine d'inflammation, quel que soit l'ordre ou le mode d'injection, on considère d'abord l'ordre ou le mode d'injection qui situe le point de mélange intermédiaire hors de la zone inflammable; puis, on met en oeuvre la deuxième étape;
- deuxième étape : comparaison, pour l'ordre ou le mode d'injection considéré, entre la réactivité des mélanges et les performances des mélangeurs ou la performance du mélangeur utilisé au cours de la phase ou de l'étape qui impose de traverser le domaine d'inflammabilité, comme déjà décrit ci-dessus. On déduit de cette comparaison le caractère inflammable du mélange en cours de formation, avec un risque maîtrisé, ou pas.

Selon l'invention, on peut donc associer à la réalisation du mélange, et même à chaque étape de sa réalisation, un risque d'inflammabilité. Ce risque peut être :
- nul, notamment si la zone d'inflammabilité du diagramme ternaire n'est pas traversée,
- ou maîtrisé, notamment si le temps de mélange est inférieur au temps d'induction chimique du mélange,
- ou pas maîtrisé du tout, en particulier si le temps de mélange est supérieur au temps d'induction chimique du mélange ou si un mélange intermédiaire est constitué, dont la composition se situe dans la zone d'inflammabilité du mélange.

On cherche en fait à déterminer le mode d'injection des gaz qui minimise ce risque. Dans certains cas, le risque ne peut être annulé ou rendu acceptable. Il faut alors modifier le mélange des gaz pour pouvoir proposer un autre mode d'injection des gaz.

Pour chaque mode, on peut attribuer un degré de risque (nul, ou acceptable, ou élevé) en fonction du plus haut degré de risque obtenu pour chacune de ses étapes ou égal à ce plus haut degré de risque. Par exemple, si la constitution d'un mélange selon un certain mode comporte une étape à risque élevé, on attribuera à l'ensemble du mode le risque élevé. Si, au contraire, une étape présente un risque nul et une autre étape un risque acceptable, l'ensemble du mode sera qualifié d'acceptable.

L'utilisation du diagramme ternaire permet de connaître l'évolution de la composition du mélange, et donc d'avoir une information importante sur le risque associé à chaque étape de la constitution du mélange.

II peut être ensuite procédé à la réalisation de l'installation pour laquelle l'ordre d'injection des gaz a pu être déterminé selon un procédé tel que décrit ci-dessus.

Une telle installation comporte notamment des canalisations d'amenée des gaz et des mélangeurs (par exemple: des injecteurs) disposés de manière à mélanger les gaz selon l'ordre de mélange déterminé ci-dessus.

L'invention permet également d'effectuer des mélanges à température élevée.

Les temps de mélange varient, en général, d'environ 100 millisecondes à plusieurs centaines de millisecondes, par exemple de 100 à 500 millisecondes comme dans le cas, déjà cité ci-dessus, de l'injecteur décrit dans le brevet US 5 356 213.

Les temps d'induction chimique peuvent, quant à eux, varier selon la nature du combustible, entre plusieurs minutes (notamment dans les conditions ambiantes de température et de pression) jusqu'à des valeurs aussi faibles que quelques dizaines de milliseconde (et ceci, en général, à haute température, par exemple aux alentours de 500°C-600°C). Des exemples de tels délais d'autoinflammation ont déjà été donnés ci-dessus, pour de l'heptane et de l'hydrogène stoechiométrique dans l'air.

Il existe donc, pour chaque mélange de gaz et chaque mélangeur donnés, une température limite en dessous de laquelle le temps de mélange est inférieur aux délais d'autoinflammation. Cette température limite définit donc une zone de sécurité au-dessus de laquelle les cinétiques deviennent très rapides, le mélange présentant alors des risques d'inflammation.

Pour un mélange donné, en connaissant l'évolution des délais d'autoinflammation ou du délai d'induction chimique en fonction de la température, il est possible de calculer cette température limite, à laquelle le délai d'autoinflammation devient de l'ordre de grandeur du, ou égal au, temps de mélange.

Un exemple particulier de mise en oeuvre de l'invention concerne un procédé chimique à boucle.

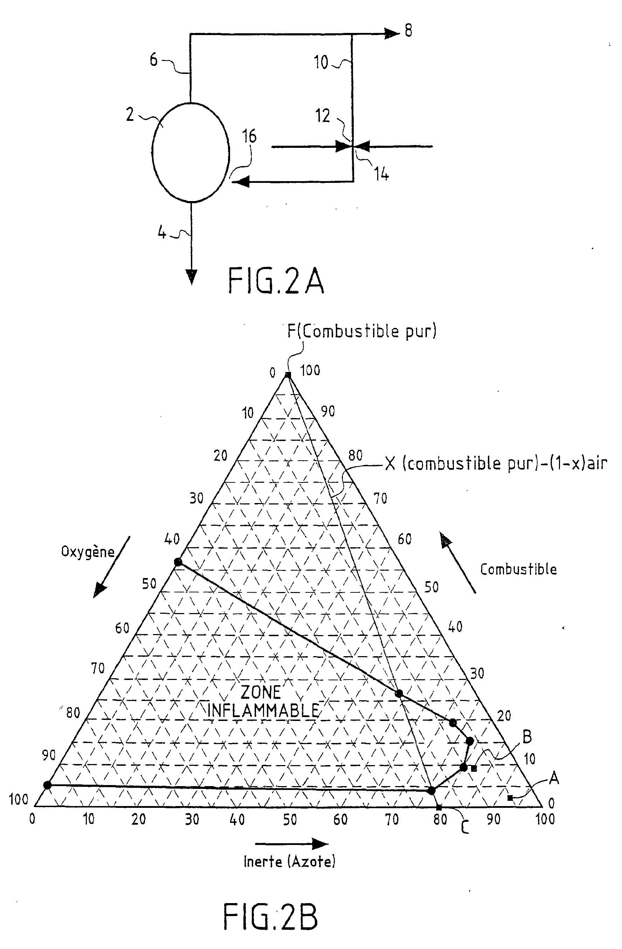

Un tel procédé est représenté schématiquement sur la figure 2A. Sur cette figure, la référence 2 désigne un réacteur, dont l'entrée 16 est alimentée avec un mélange d'un gaz combustible, d'un oxydant, et d'un gaz de recycle.

Le réacteur 2 produit un produit 4, ainsi qu'un gaz 6, partiellement éliminé par une purge 8. Ce gaz 6, composé de réactifs non réagis, de sous-produits (CO) et d'inertes (N₂, H₂O...), contient donc une certaine proportion de combustible et d'oxydant qu'il convient de recycler dans une voie 10 de recycle.

Sur le trajet de recycle, l'oxydant et le combustible sont respectivement injectés en deux points de mélange 12, 14. Un mélangeur, en chacun de ces points 12, 14, effectue ce mélange. Le mélange final alimente le réacteur 2.

Un tel schéma de recycle ne sépare pas le combustible et le gaz oxydant.

En d'autres termes, un mélange est injecté à l'entrée 16 du réacteur, ce mélange contenant de l'oxydant, du combustible, et du gaz de recycle.

La composition du gaz injecté à l'entrée 16 du réacteur, et les conditions dans lesquelles ce mélange est injecté, sont choisies de manière à réduire tout risque d'inflammabilité.

La figure 2B représente un diagramme ternaire combustible-oxydant-gaz inerte (recycle).

Sur ce diagramme, le point A correspond à la composition du mélange de gaz de recycle ou du mélange en sortie du réacteur, avant injection de l'oxydant et des combustibles. Ce mélange est constitué essentiellement de gaz inertes (à environ 92 % pour l'exemple donné), auxquels sont mélangés le gaz combustible et le gaz oxydant dans de faibles proportions (environ 3 % pour le combustible et 5 à 6 % pour l'oxydant).

Le mélange initial constitutif du gaz de recycle est donc situé en dehors de la zone inflammable du diagramme d'inflammabilité.

Le point B représente la composition du mélange injecté à l'entrée 16 du réacteur. On voit que ce point B est lui aussi situé en dehors de la zone d'inflammabilité. Il correspond à un mélange gaz inerte-combustible-oxydant plus riche en combustible et en oxydant que le mélange représenté par le point A. Par exemple, le point B correspond à une composition d'environ 82 % de gaz inerte pour environ 9 % de gaz combustible et environ 9 % d'oxydant.

Par conséquent, dans cette situation, aussi bien le gaz de recycle qui provient du réacteur 2, que le mélange injecté en entrée du réacteur, sont situés en dehors de la zone d'inflammabilité, et on pourrait penser a priori que tout danger d'inflammabilité est écarté.

La ligne combustible-air (F-C) de la figure 2B représente un ensemble de compositions pour lequel le mélange est constitué de x % de combustible dans (1-x) % d'air, x variant de 0 à 1. Ce mélange est inflammable lorsque x varie de 0,04 à 0,26. Même si le mélange final est en dehors de cet intervalle, la composition, lors de la formation du mélange, traverse la zone inflammable pendant une certaine durée. Il en est ainsi pour tout mélange de deux gaz de composition fixée. Même si les deux gaz et le point final de mélange sont hors de la zone inflammable, cette zone peut être traversée pendant la phase de mélange et présenter un danger.

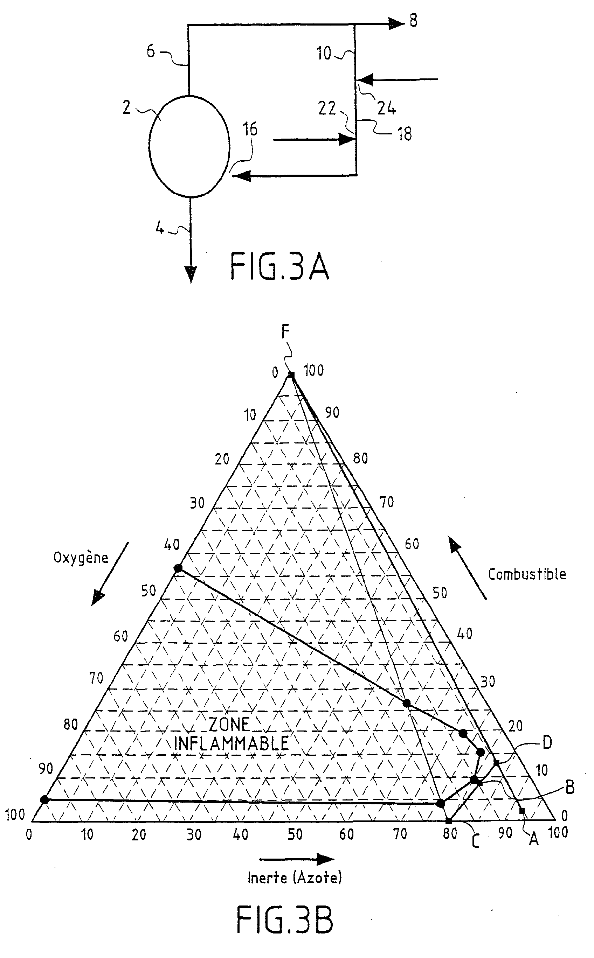

La figure 3A représente un schéma de recycle sur lequel des références identiques à celles de la figure 2A désignent des éléments identiques ou correspondants. Ce schéma diffère de celui de la figure 2A par l'ordre d'injection des gaz combustibles et oxydants. Il est d'abord procédé à l'injection du combustible, au point 24, ce qui forme temporairement un premier mélange gaz de recycle-combustible 18. Le gaz oxydant n'est injecté qu'après, au point 22, pour fournir un mélange combustible-oxydant-gaz de recycle à l'entrée 16 du réacteur.

Dans le diagramme d'inflammabilité de la figure 3B, le point de départ (composition de gaz de recycle) correspond au point A, comme sur la figure 2B.

De même, la composition du mélange en entrée du réacteur 2 est toujours identifiée par le point B, avec les mêmes compositions que déjà indiquées ci-dessus en liaison avec la figure 2B.

L'injection de gaz combustible pur (composition représentée par le point F), au point 24, fait évoluer la composition du mélange du point A au point D, c'est-à-dire à une composition d'environ 83 % de gaz inerte, 12 % de gaz de combustible et 5 % de gaz oxydant.

L'introduction, au point 22, du gaz oxydant (composition représentée par le point C (air)) fait ensuite évoluer la composition du mélange du point D au point B, selon la ligne D-C.

On constate sur ce diagramme que, dans ce cas, aussi bien les compositions initiale et finale que les compositions du mélange ou des mélanges intermédiaire(s) lors de la formation du mélange restent en dehors de la zone inflammable.

La solution qui consiste à injecter d'abord le gaz combustible au point 24, puis le gaz oxydant au point 22, est donc une solution totalement sûre. Il n'existe aucun risque d'inflammabilité en procédant ainsi.

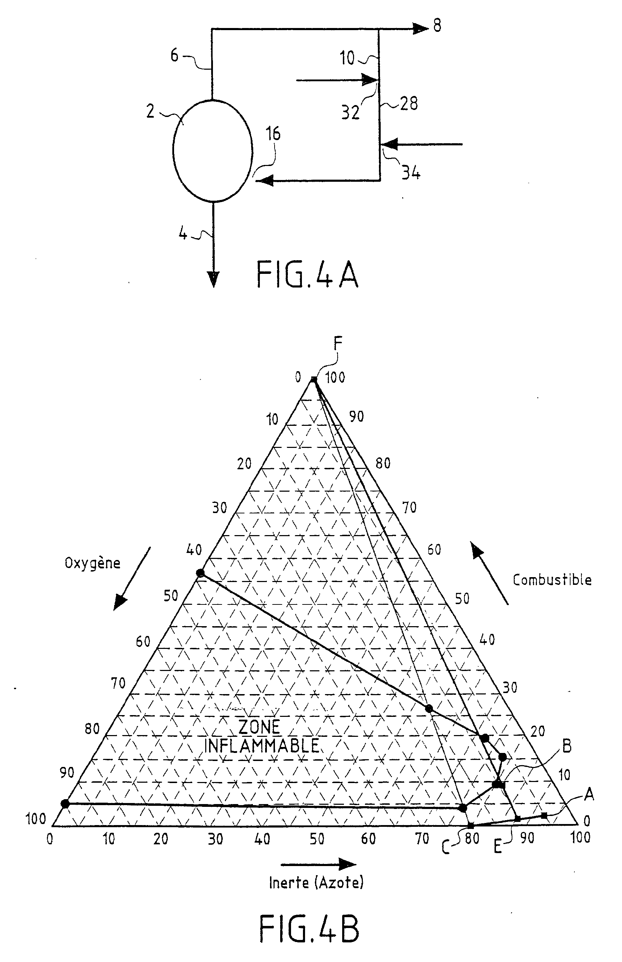

La figure 4A représente un schéma de recycle similaire au schéma de la figure 2A. Des références numériques identiques désignent des éléments similaires ou correspondants. Sur ce schéma, le gaz oxydant 32 est d'abord injecté dans le gaz de recycle 10, pour constituer un premier mélange 28 gaz inerte-gaz oxydant. Puis, le gaz combustible est injecté au point 34, pour constituer le mélange final introduit au point 16 dans le réacteur 2.

Dans le diagramme d'inflammabilité, ce schéma fait d'abord passer la composition du point A au point E (environ 88 % de gaz inerte, 10 % d'oxydant, et 2 % de gaz combustible) puis au point B, de composition déjà indiquée ci-dessus.

Initialement, lors de son injection au point 34, le gaz combustible est pur (sa composition est donnée par le point F, au sommet du diagramme d'inflammabilité). Pour atteindre le point B, le mélange doit donc traverser la zone d'inflammabilité. Cette solution, pour mélanger les gaz, présente donc un risque d'inflammabilité.

La comparaison des deux diagrammes d'inflammabilité des figures 3B et 4B montre que c'est la solution de la figure 3A, qui consiste à injecter d'abord le combustible pour mélanger ce dernier avec le gaz de recycle, puis à injecter l'oxydant pour constituer le mélange final, qui est beaucoup plus sûre que la deuxième solution. C'est donc la première solution qui doit être retenue pour effectuer un tel mélange. C'est dans l'ordre prescrit par cette première solution que les mélangeurs seront disposés, aux points 22 et 24 de la figure 3A, pour réaliser l'installation.

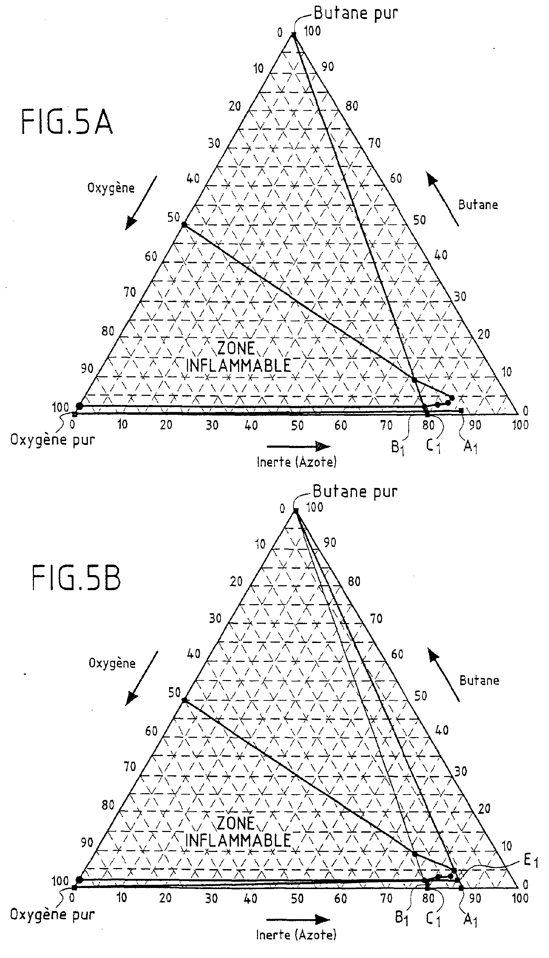

Les figures 5A et 5B représentent toutes deux le diagramme d'inflammabilité du système ternaire butane-oxygène-azote. Il s'agit du système ternaire que l'on rencontre par exemple lors de la mise en oeuvre du procédé à recycle d'oxydation du butane dans la synthèse d'anhydride maléique, décrit par exemple dans "Maleic anhydride", SRI International, Report n° 46C, octobre 1989.

Sur la figure 5A, le gaz de recycle présente initialement la composition identifiée par le point A₁ (environ 88 % d'azote et 12 % d'oxygène).

A l'entrée du réacteur, la composition, imposée par la cinétique et par le fonctionnement du réacteur, est représentée par le point B₁.

Le gaz de recycle est d'abord mélangé à de l'oxygène pur pour atteindre la composition identifiée par le point C₁. Lors de la réalisation de ce premier mélange, la composition gazeuse se trouve donc hors de la zone inflammable, et le mélange ainsi constitué ne présente aucun risque d'inflammabilité, même en présence d'une source d'énergie.

Le premier mélange ainsi obtenu est ensuite mélangé à du butane pur, jusqu'à atteindre la composition voulue B₁. L'injection de butane pur impose, comme on le voit sur le chemin de la figure 5A, la traversée de la zone inflammable, jusqu'à l'obtention de la composition voulue B₁ imposée à l'entrée du réacteur.

Au contraire, sur la figure 5B, le gaz de recycle A₁ est d'abord mélangé au butane pur, puis à l'oxygène. La composition du mélange, dans le diagramme d'inflammabilité, passe donc du point A₁ au point E₁ (la ligne qui passe par A₁ et le sommet du diagramme (butane pur) ne coupe pas la zone d'inflammabilité) puis du point E₁ au point B₁, cette fois encore sans traverser la zone d'inflammabilité.

Ce second schéma de mélange présente donc un degré de sécurité beaucoup plus élevé que celui de la figure 5A. Il correspond à un schéma de recycle du type de celui de la figure 3A, dans lequel le combustible (ici : le butane) est d'abord injecté ou mélangé avec un injecteur ou un mélangeur au point 24, pour constituer un mélange 18 de composition correspondant au point E₁ ; puis, l'oxydant est injecté ou mélangé avec un injecteur ou un mélangeur au point 22, pour constituer le mélange devant être injecté ou mélangé dans le réacteur 2.

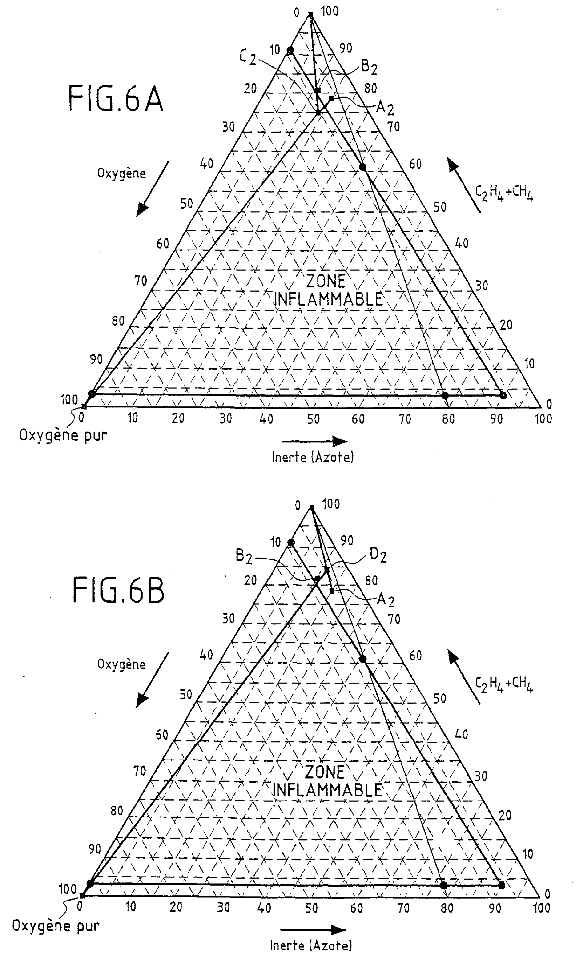

Les figures 6A et 6B représentent le diagramme d'inflammabilité du système ternaire (éthylène + méthane)-oxygène-azote. Ce système se rencontre lors de la mise en oeuvre du procédé à recycle d'oxydation de l'éthylène dans la synthèse d'oxyde d'éthylène, comme décrit dans "Ethylene-oxide and éthylène glycol", SRI consulting, Report n° 2F, janvier 1997.

Dans le schéma de la figure 6A, le gaz de recycle (de composition initiale A₂) est d'abord mélangé à de l'oxygène pur. Ce schéma de mélange est celui de la figure 4A. Le premier mélange 28 a une composition identifiée dans le diagramme par le point C₂ (environ 13 % de gaz inerte, 13 % d'oxygène, et 74 % de combustible). Ce point C₂ est situé à l'intérieur de la zone inflammable. Ceci signifie qu'avant l'injection du gaz combustible (éthylène + méthane) un premier mélange est constitué, qui présente des risques d'inflammabilité. Ce n'est que lors de la constitution du mélange final que la composition sort de la zone inflammable, pour atteindre le point B₂ (environ 10 % d'oxygène, 12 % d'azote et 78 % de combustible). Cette situation est très dangereuse puisqu'il s'agit non seulement de traverser la zone d'inflammabilité, mais aussi de constituer un mélange intermédiaire situé dans cette zone. Le risque d'inflammabilité de l'étape qui aboutit à la constitution de ce mélange intermédiaire, et donc le risque de tout le mode d'injection, est très élevé.

Dans le diagramme de la figure 6B, qui correspond au schéma de la figure 3A, on mélange d'abord le gaz de recycle au mélange éthylène-méthane, pour constituer un premier mélange 18, auquel on ajoute ensuite de l'oxygène pour constituer le second mélange injecté dans le réacteur 2. Dans ce cas, la composition évolue d'abord du point A₂ au point D₂ (premier mélange) sans traverser la zone d'inflammabilité. Lors de l'injection de l'oxygène, la zone d'inflammabilité est largement traversée, puisque l'on passe de l'oxygène pur au point B₂ dans le diagramme d'inflammabilité.

Cette seconde solution est néanmoins largement préférable à la première, puisqu'elle n'impose pas de réaliser un mélange dont la composition serait située à l'intérieur de la zone inflammable.

Il reste à comparer le délai d'inflammabilité du mélange au temps de traversée de la zone d'inflammabilité, ou au temps de mélange (qui est supérieur au temps de traversée), pour savoir si la seconde solution est cependant acceptable, ou pas. A la température du procédé de synthèse d'oxyde d'éthylène (160° C à l'entrée), ce délai d'auto-inflammabilité s'élevant à plusieurs minutes, la deuxième solution est très acceptable, puisque, comme on l'a déjà expliqué, le temps de mélange est, en général, de l'ordre de quelques centaines de millisecondes.

Le système réalisé aura donc la structure représentée sur la figure 3A, les mélangeurs étant disposés sur la canalisation 10 de manière à d'abord mélanger, au point 24, la recycle au mélange éthylène-méthane pour former un premier mélange, auquel on ajoute ensuite de l'oxygène avec un deuxième mélangeur situé au point 22 et constituer ainsi le mélange final.

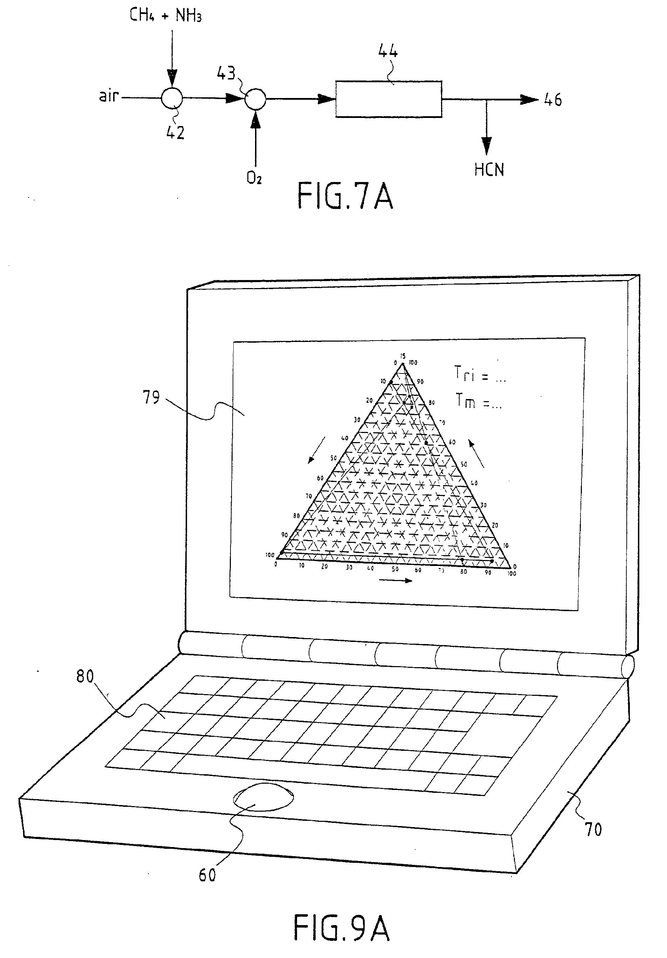

Un autre exemple de réalisation concerne le procédé de synthèse d'acide cyanhydrique, décrit notamment dans le document US 5 882 618 et qui met en oeuvre l'oxydation de l'ammoniac et du méthane. Ce procédé ne fonctionne pas avec recycle. Il est représenté schématiquement sur la figure 7A. Un mélange CH₄ et NH₃ - air-oxygène, est d'abord formé, par injection de méthane, d'ammoniac et d'oxygène dans l'air au point 42. Le réacteur 44 produit d'une part du gaz HCN et d'autre part des gaz 46 (ou « off-gas »).

Divers modes d'injection peuvent être définis :
- 1er mode : injection d'oxygène dans l'air (premier mélange), puis injection de CH₄ et NH₃ dans ce premier mélange,
- 2^{er} mode : injection de CH₄ et NH₃ dans l'air (premier mélange), puis injection d'oxygène dans ce premier mélange; c'est ce second mode qui est représenté sur la figure 7A;
- 3^{ème} mode : injection de CH₄ dans l'air (premier mélange), puis injection de NH₃ dans ce premier mélange (second mélange) et enfin injection d'oxygène dans ce second mélange,
- 4^{ème} mode : injection de NH₃ dans l'air (premier mélange), puis injection de CH₄ dans ce premier mélange (second mélange), et enfin injection d'oxygène dans ce second mélange.

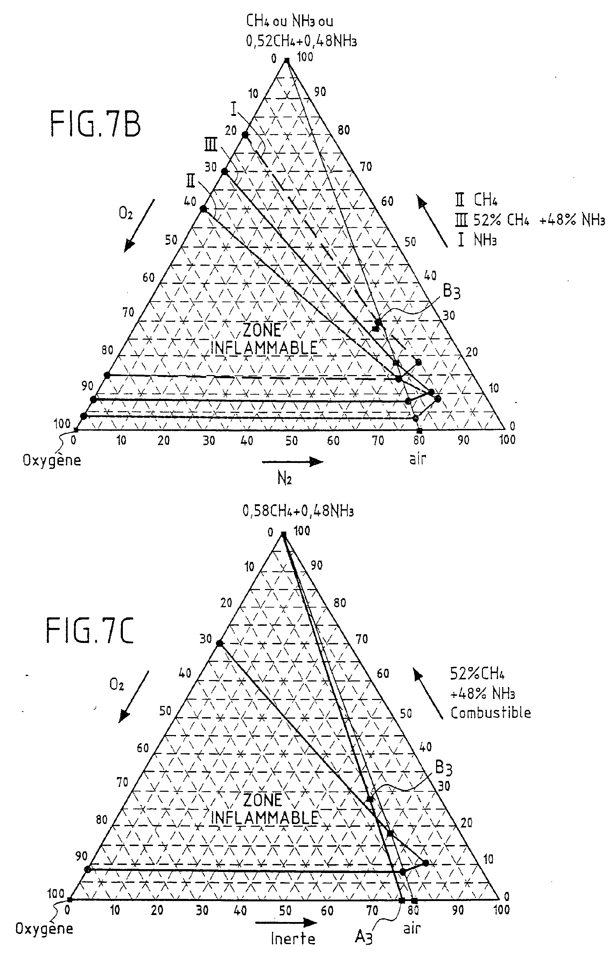

Sur la figure 7B sont représentés les domaines d'inflammabilité correspondant au mélange ammoniac-oxygène-inerte (courbe I), au mélange méthane-oxygène-inerte (courbe Il) et à un mélange (52% méthane / 48% ammoniac)-oxygène-inerte (courbe III). Sur la figure 7C est représenté uniquement le domaine d'inflammabilité du mélange (52% méthane / 48% ammoniac)-oxygène-inerte. Ce domaine peut être par exemple déterminé en utilisant la Loi Le Chatelier, qui permet de tracer les domaines d'inflammabilité à partir de mélanges de combustibles. On peut se reporter à ce sujet à Kuchta, Bulletin 680, Bureau of Mines, 1985.

Selon le premier mode d'injection, l'oxygène peut être mélangé en premier à l'air pour atteindre la composition représentée par le point A₃ (voir figure 7C). Si l'on introduit, ensuite, un mélange constitué de 52% de méthane et 48% d'ammoniac, le domaine d'inflammabilité sera traversé pour atteindre la composition finale du point opération B₃.

Si l'on injecte d'abord le mélange de combustibles à l'air (premier mélange), puis l'oxygène au premier mélange obtenu (2^{nd} mode), le domaine d'inflammabilité est traversé deux fois. Lors du deuxième mélange (mélange intermédiaire avec oxygène pur), la traversée est longue et la proportion d'oxygène dans le mélange peut être importante, et peut donc amener le mélange à être plus réactif.

La première méthode de mélange est donc meilleure.

Une comparaison des temps de réactivité du mélange et du temps caractéristique de mélangeur (supérieur au temps de traversée de la zone d'inflammabilité) confirme cette conclusion : la première méthode est de risque acceptable tandis que la deuxième est de risque plus élevé.

Si les combustibles sont injectés séparément (3^{ème} et 4^{ème} modes d'injection), on considère d'abord le diagramme ternaire azote-oxygène- 1^{er} combustible injecté, pour aboutir à un point représentatif de la composition du second mélange; puis le diagramme ternaire azote-oxygène- 2^{ème} combustible injecté est considéré, pour aboutir à un point représentatif de la composition du mélange final lorsque l'on part de la composition du second mélange.

D'une manière générale, le traitement du cas où on mélange au moins trois gaz réactifs ou combustibles à un gaz inerte ou de fond se ramène donc au traitement successif de plusieurs cas de mélange de deux gaz à un gaz inerte ou de fond.

On remarque alors, compte tenu de la position des trois points d'injection et de la composition du point opératoire final (point A₃ : 27% de combustibles (52% CH₄ et 48% NH₃), 17% d'oxygène et 56% d'azote) qu'il est plus sécuritaire de mélanger l'air et l'oxygène en premier, puis de procéder au mélange avec les combustibles. Dans ce cas, injecter les combustibles l'un après l'autre ne présente aucun avantage.

Pour ne traverser le domaine d'inflammabilité qu'une seule fois, il est préférable de réaliser un mélange air-oxygène puis combustible ; dans ce cas, il vaut mieux introduire un mélange de combustibles, et traverser brièvement le nez du domaine d'inflammabilité, plutôt que d'introduire les combustibles les uns après les autres, et traverser « deux nez » relatifs aux domaines d'inflammabilité de chacun des combustibles.

Pour la réalisation pratique du système de mélange des gaz, on inverse donc, par rapport à la figure 7A, les points d'injection de l'oxygène et du mélange CH₄ + NH₃. Au point 42 est donc disposé un mélangeur pour l'injection de l'oxygène, et au point 43 un mélangeur pour ajouter le mélange CH₄ et NH₃ au premier mélange ainsi constitué.

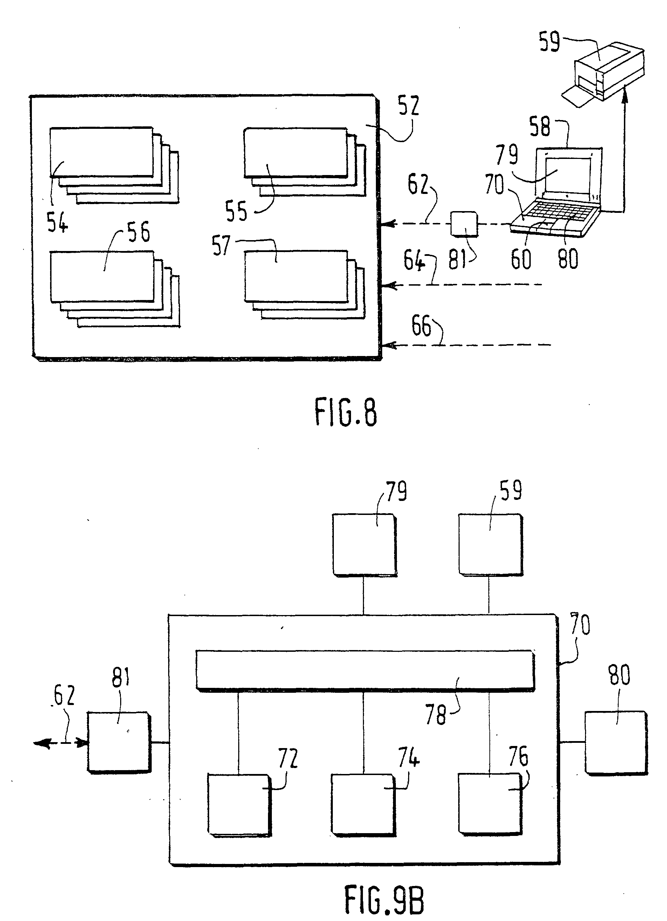

Un exemple de réalisation d'un système permettant la mise en oeuvre de l'invention va être décrit en liaison avec les figures 8 à 9B.

Sur la figure 8, la référence 52 désigne soit un ordinateur central, soit un site web. Dans le deuxième cas, ce site n'est de préférence accessible que par un réseau intranet.

Dans les deux cas, des bases de données 54, 55, 56, 57 sont hébergées dans l'ordinateur ou sur le site. De telles bases de données peuvent être mises à jour et concernent par exemple, respectivement:
- une base de données 54 dans laquelle sont recensés des diagrammes ternaires de gaz combustibles,
- une base de données 55 regroupant, pour divers gaz, ou mélanges de gaz, les temps d'induction, ou délais d'inflammation, et leur évolution en fonction de données de pression et de température,
- une base de données 56 concernant divers mélangeurs, par exemple des injecteurs, avec leur temps de mélange,
- et éventuellement une base de donnée 57 concernant du matériel, ou équipement, par exemples des détendeurs et/ou des robinets, ..., pouvant être utilisés en combinaison avec les injecteurs de la base 56 et les gaz de la base 55.

Divers utilisateurs peuvent être connectés aux moyens 52 d'hébergement de ces bases de données. Sur la figure 8, un de ces utilisateurs est représenté par son ordinateur portable ou de type PC 58, la connexion avec les moyens 52 étant assurée par un réseau de communication 62, par exemple un réseau intranet 62 et par des moyens de connexion à ce réseau (modem ou carte réseau 81).

En parallèle, d'autres utilisateurs, non représentés sur la figure, peuvent être connectés aux moyens d'hébergement 52, par des moyens de communication 64, 66, ... , qui peuvent faire partie d'un réseau: L'ordinateur de chaque utilisateur y est également connecté par un modem ou une carte réseau.

Selon une variante, les bases de données, 54, 55, 56, 57 peuvent aussi être mémorisées dans les moyens de mémorisation du dispositif informatique 58, dans la mesure où la taille de ces bases de données et la taille mémoire disponible dans ce système informatique 58 sont compatibles.

Le système informatique 58 peut être par exemple un micro-ordinateur de type PC du commerce. Il est représenté en figure 9A avec affichage d'un diagramme ternaire et d'une zone d'inflammabilité.

Il comporte (figure 9B) une unité centrale 70, qui comprend elle-même un microprocesseur 72, un ensemble 74 de mémoires ROM et RAM, un disque dur 76, qui a aussi une fonction de stockage d'informations, tous ces éléments étant couplés à un bus 78.

Un écran 79 permet de visualiser des informations sur les données rentrées dans le système par un opérateur (par exemple, le nom d'un mélange de gaz et les données de température et de pression d'utilisation de ce mélange de gaz), et les données fournies par les bases de données en réponse aux données entrées par l'opérateur (par exemple: diagramme ternaire et données sur le temps d'induction chimique d'un mélange).

Le système dispose également de périphériques de contrôle, et notamment d'un clavier 80 et d'une souris 60. D'autres moyens de sélection d'une zone ou un champ d'une page affichée à l'écran 79 peuvent être également utilisés, par exemple tout moyen permettant de réaliser une sélection par contact tactile sur l'écran.

Les instructions pour mettre en oeuvre un procédé selon l'invention sont mémorisées dans les moyens 74, 76 de mémorisation du système informatique 58. Les programmes correspondants peuvent aussi être résidents sur un site web 52 auquel un utilisateur se connecte via le terminal 58, les moyens 81 et le réseau 62.

Selon un autre mode de réalisation, toute ou partie de l'information contenue dans les bases de données 54, 55, 56, 57 est contenue dans la mémoire RAM du système informatique 58 et / ou dans le disque dur 76.

En réponse aux instructions de l'utilisateur, une connexion est établie entre le système 58 et les moyens 52 qui hébergent les bases de données et, éventuellement, les programmes à mettre en oeuvre pour exécuter le procédé. Ces programmes comportent par exemple:
- un programme de détermination de temps d'inflammation à partir des équations des cinétiques des réactions,
- un programme de détermination de domaines d'inflammabilité, par exemple à partir de la loi Le Chatelier,
- un programme comportant les instructions pour mettre en oeuvre un procédé tel que décrit ci-dessus en liaison avec la figure 1 (et pouvant donc être organisé selon l'organigramme de la figure 1) ou selon une de ses variantes décrites ci-dessus, et en particulier permettant de comparer les temps de traversée d'un, ou de, domaine(s) d'inflammabilité ou les temps de mélange et le(s) temps d'induction chimique(s).

Là encore, une partie de ces programmes peut être contenue dans la mémoire du système informatique 58 et / ou dans le disque dur 76.

Une session interactive peut alors être initialisée, au cours de laquelle, en réponse à des données introduites par l'utilisateur, le système 52 va envoyer à cet utilisateur des données sélectionnées par recherche dans les bases de données 54, 55, 56, 57.

A l'aide du système informatique 58, un mélange est sélectionné. Les informations spécifiques à ce mélange, et relatives à son diagramme ternaire, avec la zone d'inflammabilité correspondante pour des conditions de température et de pression données, et éventuellement à son temps d'induction, sont recherchées dans la base 56 et envoyées au système informatique 58, mémorisées dans les moyens de mémorisation 74, 76. Ces informations ou ces données peuvent aussi être calculées à partir des programmes mémorisés mentionnés ci-dessus. Elles peuvent être affichées sur l'écran 78. Elles peuvent être aussi validées et corrigées à partir de cet affichage.

Puis, un mélangeur (par exemple : un injecteur) est sélectionné. Les informations spécifiques à ce mélangeur, et relatives en particulier à son temps de mélange, sont recherchées dans la base 56 (ou éventuellement calculées) et envoyées au système informatique 58, puis mémorisées dans les moyens de mémorisation 74, 76. Ces informations peuvent être affichées sur l'écran 78. Elles peuvent être aussi validées et corrigées.

Toutes les données ainsi rassemblées sont conservées dans les mémoires 74, 76 du système informatique 58. Elles peuvent aussi être envoyées à l'ordinateur central 52.

Des données peuvent aussi être introduites dans le système 58, par exemple à la main à l'aide du clavier 80 et sans recours à une base de données particulière. Ces données sont elles aussi mémorisées, dans les mémoires 74, 76 du système 58, ou envoyées vers le système 52.

Le diagramme ternaire ainsi que les diverses données sur les temps d'induction et de mélange peuvent être affichés sur l'écran 78.

Le système 58 (ou les moyens 52) peut incorporer un logiciel de calcul pour calculer un temps d'induction de manière numérique, à partir de données fournies par l'utilisateur. Il peut également incorporer un logiciel de calcul pour calculer des diagrammes ternaires et leur domaine d'inflammabilité à différentes températures et/ou pressions.

Les instructions des programmes pour mettre en oeuvre un procédé selon l'invention sont mémorisées dans une zone mémoire du système informatique 58 ou du système 52. Ces instructions sont par exemple installées à partir d'un support pouvant être lu par le système informatique 58 et sur lequel elles sont enregistrées. Un tel support peut être par exemple un disque dur, une mémoire morte ROM, un disque optique compact, une mémoire vive dynamique DRAM ou tout autre type de mémoire RAM, un élément de stockage magnétique ou optique, des registres ou d'autres mémoires volatiles et/ou non volatiles.

## Revendications

1. Procédé pour déterminer le risque d'inflammabilité du mélange d'au moins deux gaz réactifs A, B, dans un gaz inerte ou de fond, ou l'ordre de mélange de ces gaz réactifs dans le gaz inerte ou de fond, caractérisé en ce qu'il comporte
- une étape pour déterminer si la composition du mélange traverse, pendant sa formation, la zone d'inflammabilité dans le diagramme ternaire du mélange A-B-gaz inerte ou de fond, lorsque ce mélange est réalisé suivant un premier mode selon lequel A est d'abord mélangé dans le gaz inerte ou de fond pour former un premier mélange, puis B est mélangé dans le premier mélange pour former le mélange final.

2. Procédé selon la revendication 1, comportant en outre:
- une étape de détermination d'un premier temps, de traversée de la zone d'inflammabilité dudit diagramme ternaire, lorsque ce mélange est réalisé suivant le premier mode,
- une étape de comparaison de ce premier temps de traversée au temps d'induction chimique du mélange ou du mélange stoechiométrique.

3. Procédé selon la revendication 1, comportant en outre une étape de comparaison d'un, ou des, temps de mélange du, ou des, mélangeur(s) utilisé(s), au temps d'induction chimique du mélange ou du mélange stoechiométrique.

4. Procédé selon l'une des revendications 1 à 3, comportant en outre, si la composition du mélange traverse la zone d'inflammabilité dudit diagramme ternaire, ou bien si le premier temps de traversée ou le temps de mélange est supérieur au temps d'induction chimique du mélange:
- une étape pour déterminer si la composition du mélange traverse, pendant sa formation, la zone d'inflammabilité dans le diagramme ternaire du mélange A-B-gaz inerte ou de fond, lorsque celui-ci est réalisé suivant un second mode selon lequel B est d'abord mélangé dans le gaz inerte ou de fond pour former un premier mélange, puis A est mélangé dans le premier mélange pour former le mélange final.

5. Procédé selon la revendication 4, comportant en outre
- une étape de détermination d'un second temps, de traversée de la zone d'inflammabilité dudit diagramme ternaire, lorsque ce mélange est réalisé suivant le second mode,
- une étape de comparaison de ce second temps de traversée au temps d'induction chimique du mélange ou du mélange stoechiométrique.

6. Procédé selon la revendication 4, comportant en outre une étape de comparaison d'un, ou des, temps de mélange du, ou des, mélangeur(s) utilisé(s), au temps d'induction chimique du mélange ou du mélange stoechiométrique.

7. Procédé selon l'une des revendications 1 à 6, comportant, si la composition de l'un des mélanges selon l'un des modes d'injection ne traverse pas la zone d'inflammabilité pendant sa formation, la sélection de ce mode.

8. Procédé selon l'une des revendications 2 ou 3 ou 5 ou 6, comportant la sélection d'un mode ou du mode pour lequel le, ou les, temps de mélange ou le temps de traversée de la zone d'inflammabilité dudit diagramme ternaire est inférieur au temps d'induction chimique du mélange.

9. Procédé selon l'une des revendications 1 à 6, comportant, si les deux modes d'injection imposent tous deux de traverser le domaine d'inflammabilité, la sélection du mode pour lequel:
(i) - le point de mélange intermédiaire, représentatif de la composition du premier mélange, est situé en dehors de la zone d'inflammabilité;
(ii) - le, ou les, temps de mélange ou le temps de traversée de la zone d'inflammabilité du diagramme ternaire est inférieur au temps d'induction chimique du mélange.

10. Procédé de réalisation d'un mélange d'au moins deux gaz réactifs A, B, dans un gaz inerte ou de fond, comportant :
- la détermination du risque d'inflammabilité du mélange, lors de la réalisation du mélange, ou de l'ordre de mélange de ces gaz réactifs dans le gaz inerte ou de fond, selon l'une des revendications 1 à 9,
- le mélange des gaz réactifs A et B, dans l'ordre pour lequel la composition du mélange ne traverse pas la zone d'inflammabilité lors de son évolution, ou pour lequel le, ou les, temps de mélange ou le temps de traversée de la zone d'inflammabilité du diagramme ternaire est inférieur au temps d'induction chimique du mélange.

11. Procédé de réalisation d'un mélange final d'au moins deux gaz réactifs A, B, dans un gaz inerte ou de fond, caractérisé en ce qu'il comporte :
- le mélange du gaz A dans le gaz inerte ou de fond, pour former un premier mélange, dont la composition évolue, au cours de sa formation, en dehors de la zone d'inflammabilité du diagramme ternaire du mélange A-B-gaz inerte, ou traverse la zone d'inflammabilité avec un, ou des, temps de mélange du, ou des, mélangeur(s) utilisé(s), ou un temps de traversée de la zone d'inflammabilité, inférieur au temps d'induction chimique du premier mélange,
- le mélange du gaz B, dans le premier mélange, en formant un mélange dont la composition évolue vers celle du mélange final, la composition de ce mélange évoluant, dans ledit diagramme ternaire, soit sans traverser la zone d'inflammabilité soit en traversant la zone d'inflammabilité, avec un, ou des, temps de mélange du, ou des, mélangeur(s) utilisé(s), ou un temps de traversée de la zone d'inflammabilité, inférieur au temps d'induction chimique du mélange.

12. Procédé selon l'une des revendications 1 à 11, comportant une étape préalable de détermination du, ou des, temps de mélange d'un, ou des, mélangeur(s) destiné(s) à être utilisé(s) pour réaliser le mélange des gaz réactifs, et de la température à laquelle le délai d'autoinflammation du mélange devient égal ou sensiblement égal à l'un des temps de mélange.

13. Procédé selon l'une des revendications 1 à 12, le mélange étant à réaliser à une température comprise entre 300°C et 600°C.

14. Procédé selon l'une des revendications 1 à 13, le mélange des deux gaz réactifs A, B, dans un gaz inerte ou de fond, étant celui d'un procédé de recycle.

15. Procédé selon l'une des revendications 1 à 13, le mélange des gaz réactifs étant un mélange d'oxygène et de butane dans un gaz inerte.

16. Procédé selon l'une des revendications 1 à 13, le mélange des gaz réactifs étant un mélange d'oxygène et d'éthylène dans un gaz inerte.

17. Procédé selon l'une des revendications 1 à 13, les gaz réactifs à mélanger dans le gaz inerte ou de fond étant au nombre de trois au moins, et l'ordre de mélange des gaz étant déterminé en considérant les couples de gaz pouvant être mélangés successivement, et les diagrammes ternaires correspondants.

18. Procédé selon l'une des revendications 1 à 17, comportant une étape de consultation d'une base de données électronique comportant des données sur des diagrammes ternaires et/ou la consultation d'une base de données électronique comportant des données sur des temps d'induction de mélanges de gaz et/ou la consultation d'une base de données électronique comportant des données sur des temps de mélange de mélangeurs.

19. Procédé selon l'une des revendications 1 à 18, comportant en outre une représentation graphique, sur un écran de visualisation, du ou des diagramme(s) ternaire(s) considéré(s) et de la, ou des, zone(s) d'inflammabilité correspondante(s) dans ce, ou ces, diagramme(s).

20. Procédé de réalisation d'une installation pour mélanger au moins deux gaz réactifs A, B, dans un gaz de fond, comportant les étapes suivantes:
- on détermine l'ordre de mélange de ces gaz, selon l'une des revendications 1 à 19,
- on réalise une installation, de manière à mélanger les gaz selon l'ordre ainsi déterminé.

21. Dispositif pour établir le risque d'inflammabilité de mélanges de gaz, chaque mélange comportant au moins deux gaz réactifs A, B, dans un gaz inerte ou de fond, ou pour déterminer l'ordre de mélange de ces gaz réactifs dans le gaz inerte ou de fond, caractérisé en ce qu'il comporte :
- des moyens (52) mémorisant au moins une base de données (54) comportant, pour des mélanges de gaz, des données sur les diagrammes ternaires de ces mélanges, et les zones d'inflammabilité dans ces diagrammes pour des conditions de température et de pression données,
- des moyens (80) pour sélectionner un mélange de gaz et des conditions de pression et de température d'utilisation de ce mélange de gaz,
- des moyens de visualisation (79) d'un diagramme ternaire et de la zone d'inflammabilité d'un mélange dans ce diagramme.

22. Dispositif selon la revendication 21, comportant en outre des moyens (72, 74) pour calculer, ou spécialement programmés pour calculer ou établir, pour des mélanges de gaz comportant chacun au moins deux gaz réactifs A, B, dans un gaz inerte ou de fond, et pour des conditions de température et de pression de ces mélanges, une zone d'inflammabilité dans le diagramme ternaire du mélange.

23. Dispositif selon la revendication 21 ou 22, comportant des moyens (72, 74) pour calculer, ou spécialement programmés pour calculer ou établir, pour des mélanges de gaz comportant chacun au moins deux gaz réactifs A, B, dans un gaz inerte ou de fond, et pour des conditions de température et de pression de ces mélanges, un temps de traversée, par ce mélange, de la zone d'inflammabilité du diagramme ternaire correspondant.

24. Dispositif selon l'une des revendications 21 à 23, comportant des moyens (52) mémorisant au moins une base de données (55) comportant, pour des mélanges de gaz, des données sur les temps d'induction, ou délais d'inflammations, de ces mélanges, en fonction de conditions de température et de pression.

25. Dispositif selon l'une des revendications 21 à 24, comportant des moyens (22, 24) pour calculer, ou spécialement programmés pour calculer ou établir, pour des mélanges de gaz comportant chacun au moins deux gaz réactifs A, B, dans un gaz inerte ou de fond, et en fonction de conditions de température et de pression de ces mélanges, des temps d'induction chimique de ces mélanges.

26. Dispositif selon l'une des revendications 21 à 25, comportant en outre une base de données (56) comportant des informations sur des temps de mélange de mélangeurs, et des moyens (80) pour sélectionner un mélangeur.

27. Dispositif selon l'une des revendications 21 à 26, comportant en outre des moyens (72, 74) pour, ou spécialement programmés pour, comparer un temps de mélange ou un temps de traversée d'une zone d'inflammabilité d'un des diagrammes ternaires, et un temps d'induction chimique de ce mélange.

28. Dispositif pour établir le risque d'inflammabilité de mélanges qui sont chacun constitués d'au moins deux gaz réactifs A, B dans- un gaz inerte ou de fond, ou pour établir l'ordre de mélange de ces gaz réactifs dans le gaz inerte ou de fond, caractérisé en ce qu'il comporte des moyens pour calculer ou spécialement programmés pour calculer, en fonction de conditions de température et de pression:
- le diagramme ternaire d'un mélange et la zone d'inflammabilité dans ce diagramme,
- un temps d'induction chimique de ces mélanges.

29. Dispositif selon la revendication 28, comportant en outre des moyens (79) pour visualiser un diagramme ternaire et la zone d'inflammabilité d'un mélange dans ce diagramme.

30. Dispositif selon l'une des revendications 28 ou 29, comportant en outre des moyens pour calculer un temps de mélange d'un mélangeur donné ou pour mémoriser des temps de mélange d'un ensemble de mélangeurs.

31. Dispositif terminal (58) pour établir le risque d'inflammabilité du mélange d'au moins deux gaz réactifs A, B, dans un gaz inerte ou de fond, ou l'ordre d'injection de ces gaz réactifs dans le gaz inerte ou de fond, caractérisé en ce qu'il comporte :
- des moyens de communication (62, 64, 66, 81) pour établir une communication entre ledit dispositif terminal et des moyens (52) contenant au moins une base de données (54, 55, 56, 57) comportant, pour des mélanges de gaz, des données sur les diagrammes ternaires de ces mélanges, et les zones d'inflammabilité dans ces diagrammes en fonction des conditions de température et de pression,
- des moyens (80) pour fournir audit terminal des données d'utilisateur dudit terminal, comportant au moins un mélange de gaz utilisé, et les conditions de pression et de température d'utilisation,
- des moyens de mémorisation (74, 76), en communication avec les moyens pour fournir audit terminal des données d'utilisateur, pour mémoriser ces données d'utilisateur, ainsi que des données fournies par la base de données sur le diagramme ternaire du mélange sélectionné,
- des moyens d'affichage (79), en communication avec les moyens de mémorisation, pour représenter graphiquement au moins le diagramme ternaire fourni par la base de données.

32. Programme d'ordinateur comportant les instructions pour mettre en oeuvre un procédé selon l'une des revendications 1 à 19.

33. Support de données, pouvant être lu par un système informatique, comportant les données, sous forme codée, pour mettre en oeuvre un procédé selon l'une des revendications 1 à 19.

34. Produit logiciel comportant un moyen de support de données de programme, susceptible d'être lu par un système informatique, permettant de mettre en oeuvre un procédé selon l'une des revendications 1 à 19.
